# EUROPÄISCHE PATENTANMELDUNG

(11) **EP 4 819 051 A1**
(43) Veröffentlichungstag der Anmeldung: **07.10.2026**
(21) Anmeldenummer: 25167437.0
(22) Anmeldetag: 31.03.2025
(51) Int. Cl.: G06T 11/00

(54) **VERFAHREN ZUM VERARBEITEN EINES MULTISPEKTRALEN BILDDATENSATZES**

(71) Anmelder: Siemens Healthineers AG, 91301 Forchheim (DE)
(72) Erfinder: Dr. Raupach, Rainer, 91336 Heroldsbach (DE)
(74) Vertreter: Siemens Healthineers Patent Attorneys

(57) **Zusammenfassung**

Die Erfindung betrifft ein Verfahren zum Verarbeiten eines Bilddatensatzes, welcher auf einer multispektralen Computertomographie-Bildgebung basiert und welcher ein erstes Bildwerte-Tupel und ein zweites Bildwerte-Tupel aufweist, wobei das erste Bildwerte-Tupel einem ersten Volumenelement eines abzubildenden Bereichs zugeordnet ist, wobei das zweite Bildwerte-Tupel einem zweiten Volumenelement des abzubildenden Bereichs zugeordnet ist, das Verfahren umfassend:
- ein Berechnen eines ersten Anteils eines Basismaterials, indem eine Abbildung auf das erste Bildwerte-Tupel angewendet wird, wobei der erste Anteil des Basismaterials dem ersten Volumenelement des abzubildenden Bereichs zugeordnet ist, wobei die Abbildung an dem ersten Bildwerte-Tupel eine erste Steigung aufweist, wobei die erste Steigung ungleich Null ist,
- ein Berechnen eines zweiten Anteils des Basismaterials, indem die Abbildung auf das zweite Bildwerte-Tupel angewendet wird, wobei der zweite Anteil des Basismaterials dem zweiten Volumenelement des abzubildenden Bereichs zugeordnet ist, wobei die Abbildung an dem zweiten Bildwerte-Tupel eine zweite Steigung aufweist, wobei die zweite Steigung ungleich Null und ungleich der ersten Steigung ist.

## Beschreibung

Die Erfindung betrifft ein Verfahren zum Verarbeiten eines Bilddatensatzes, welcher auf einer multispektralen Computertomographie-Bildgebung basiert.

Metalle verursachen in Computertomographie-Bildern in der Regel Artefakte, die diagnostisch relevante Bildbereiche überdecken. Die physikalischen Ursachen dieser Artefakte sind vielfältig und können je nach Art und Form des Metalls von Strahlaufhärtung, Abtastfehlern an Kanten, Streustrahlung oder Signalauslöschung dominiert sein. Beispielsweise verursachen Metalle mit kleinen Kernladungszahlen und Dichten, wie z.B. Aluminium oder Titan, Artefakte in erster Linie aufgrund von Strahlaufhärtung.

Auch diverse weitere Formen von Artefakten sind in der Bildgebung grundsätzlich ein Problem, für die Computertomographie (CT) insbesondere Ringartefakte. Dies liegt daran, dass Signalfehler eines Kanals sich durch die CT-Rekonstruktion in ringartige Störungen übersetzen. Wie jede Art von Artefakten beeinträchtigen sie die diagnostische Qualität von CT-Bildern.

In der spektralen CT kommt erschwerend hinzu, dass Artefakte in den ursprünglich aufgenommen spektralen Informationen ("primäre spektrale Daten") zwar nicht signifikant sind, beispielsweise weil sie ein genügend kleines Signal-zu-Rausch-Verhältnis aufweisen und deswegen nicht wahrnehmbar sind, bei Ableitung von spektralen Ergebnissen wie monoenergetischen oder Basismaterialbildern jedoch sichtbar werden.

Im Hinblick auf Strahlaufhärtungsartefakte von Metallen sind verschiedene Verfahren zur Artefakt-Korrektur bekannt. Bei segmentierungsbasierten Verfahren werden durch Segmentierung mithilfe einer Schwelle die Metallbereiche identifiziert und dann wird ausgehend von der lokalen Schwächung ein Rückschluss auf den relevanten Metallanteil gezogen, der wiederum eine Abschätzung des Datenfehlers durch Strahlaufhärtung ermöglicht. Dies erfordert jedoch in der Regel eine Hypothese, um welches Metall es sich handelt, und ist damit nicht ohne Weiteres für beliebige Metalle und Legierungen anwendbar.

Wenn spektrale Information verfügbar ist, beispielsweise in einer Dual Energy- oder Multi-Energy-CT, dann können durch Subtraktion der spektralen Kanäle Strahlaufhärtungsartefakte reduziert werden. Dabei wird etwa ein niederenergetisches Bild gewichtet von einem hochenergetischen Bild abgezogen, *I* = (1 *+ w*)*I_{Hi} - wI_{Lo}.* Da die Strahlaufhärtungsartefakte im niederenergetischen Bild i.d.R. stärker ausgeprägt sind als im hochenergetischen Bild, kompensieren sich diese bei einer geeigneten Mischung. Allerdings entspricht dieses Vorgehen nur der Extrapolation einer noch höheren Energie als *I_{Hi},* die natürlich weniger Strahlaufhärtungsartefakte aufweist. Als wesentlicher Nachteil werden deswegen hierbei alle Weichteilkontraste stark reduziert, so dass diese Lösung nur eine Scheinlösung darstellt und daher auch als Pseudo-Strahlaufhärtungskorrektur bezeichnet werden kann.

In DE 10 2016 204 709 A1 wird ein Verfahren beschrieben, mit dem bei Verfügbarkeit von spektraler Information Strahlaufhärtungsartefakte reduziert werden können. Insbesondere wird darin eine Zerlegung beschrieben, die auf einem Basismaterialsystem basiert, das genauso viele Freiheitsgrade aufweist, wie Spektren zur Verfügung stehen, wobei angenommen wird, dass jedes andere Material sich als Linearkombination der Basismaterialien darstellen und so auch im Sinne der Strahlaufhärtung erfassen lässt.

Ebenso als Stand der Technik zur Beschreibung von Verfahren zur Strahlaufhärtungskorrektur kann EP 4 343 703 A1 betrachtet werden. Hier werden durch eine Transformation *A* kombinierte spektrale Daten statt real existierende Basismaterialien betrachtet. Im Sinne der Beschreibung der Erfindung können auch diese als "Basismaterialien" (ohne real existierendes Gegenstück) angesehen werden.

Eine Verhinderung bzw. physikalische Korrektur von Datenfehlern, insbesondere im Hinblick auf Ringartefakte, ist nicht immer in ausreichender Qualität möglich ist. Deswegen werden Artefakte oft mit heuristischen Methoden entfernt, wie beispielsweise in US 6 044 125 A. Diese Methode steht stellvertretend für das Prinzip Detektion von Artefakten, Erzeugung eines Artefakt-Bildes, das nur die Artefakte enthält, und anschließende Subtraktion des Artefakt-Bildes, was zu deren Reduktion führt.

Wenn jedoch die Signifikanz der Artefakte zu gering ist, dann arbeiten derartige Verfahren zwar ausreichend für die primären Daten, da die Artefakte aufgrund ihrer geringen Signifikanz gar nicht relevant waren, verhindern allerdings damit nicht, dass die Artefakte in aus den Primärdaten abgeleiteten Ergebnissen sichtbar werden.

Die Erfindung hat die Aufgabe eine Alternative zu bekannten Verfahren zum Verarbeiten multispektraler Computertomographie-Bilddaten bereitzustellen. Jeder Gegenstand eines unabhängigen Anspruchs löst diese Aufgabe. In den abhängigen Ansprüchen sind weitere vorteilhafte Aspekte der Erfindung berücksichtigt. Unabhängig vom grammatikalischen Geschlecht eines bestimmten Begriffes sind Personen mit männlicher, weiblicher oder anderer Geschlechteridentität mit umfasst.

Die Erfindung betrifft ein computerimplementiertes Verfahren zum Verarbeiten eines Bilddatensatzes, welcher auf einer multispektralen Computertomographie-Bildgebung basiert und welcher ein erstes Bildwerte-Tupel und ein zweites Bildwerte-Tupel aufweist, wobei das erste Bildwerte-Tupel einem ersten Volumenelement eines abzubildenden Bereichs zugeordnet ist, wobei das zweite Bildwerte-Tupel einem zweiten Volumenelement des abzubildenden Bereichs zugeordnet ist, das Verfahren umfassend:
- ein Berechnen eines ersten Anteils eines Basismaterials, indem eine Abbildung auf das erste Bildwerte-Tupel angewendet wird, wobei der erste Anteil des Basismaterials dem ersten Volumenelement des abzubildenden Bereichs zugeordnet ist, wobei die Abbildung an dem ersten Bildwerte-Tupel eine erste Steigung aufweist, wobei die erste Steigung ungleich Null ist,
- ein Berechnen eines zweiten Anteils des Basismaterials, indem die Abbildung auf das zweite Bildwerte-Tupel angewendet wird, wobei der zweite Anteil des Basismaterials dem zweiten Volumenelement des abzubildenden Bereichs zugeordnet ist, wobei die Abbildung an dem zweiten Bildwerte-Tupel eine zweite Steigung aufweist, wobei die zweite Steigung ungleich Null und ungleich der ersten Steigung ist.

Eine Ausführungsform sieht vor, dass ein zusammenhängender Teilraum der Bildwerte das erste Bildwerte-Tupel und das zweite Bildwerte-Tupel aufweist, wobei die Abbildung an jedem Bildwerte-Tupel des zusammenhängenden Teilraumes stetig ist.

Eine Ausführungsform sieht vor, dass in Bezug auf ein Maß für eine Abschwächung von Röntgenstrahlung die Abschwächung der Röntgenstrahlung durch das zweite Volumenelement stärker ist als die Abschwächung der Röntgenstrahlung durch das erste Volumenelement,
- wobei die Steigung der Abbildung an dem zweiten Bildwerte-Tupel steiler ist als an dem ersten Bildwerte-Tupel.

Eine Ausführungsform sieht vor, dass der erste Bilddatensatz ein drittes Bildwerte-Tupel aufweist, wobei das dritte Bildwerte-Tupel einem dritten Volumenelement des abzubildenden Bereichs zugeordnet ist, wobei in Bezug auf das Maß für die Abschwächung der Röntgenstrahlung die Abschwächung der Röntgenstrahlung durch das dritte Volumenelement stärker ist als die Abschwächung der Röntgenstrahlung durch das zweite Volumenelement,
- wobei eine Steigung der Abbildung an dem dritten Bildwerte-Tupel flacher ist als an dem zweiten Bildwerte- Tupel.

Eine Ausführungsform sieht vor, dass das Maß für die Abschwächung der Röntgenstrahlung ein ausgewähltes Spektrum, insbesondere das Spektrum des ersten Bildwerts der Bildwerte-Tupel, der multispektralen Computertomographie-Bildgebung betrifft.

Eine Ausführungsform sieht vor, dass das Maß für die Abschwächung der Röntgenstrahlung eine Linearkombination, insbesondere in Form einer gewichteten Mischung, von verschiedenen Spektren der multispektralen Computertomographie-Bildgebung zugeordneten Bildwerten betrifft.

Eine Ausführungsform sieht vor, dass das Maß für die Abschwächung angibt, ob die Abschwächung der Röntgenstrahlung einen vorgegebenen Schwellwert überschreitet und/oder unterschreitet.

Die Erfindung betrifft ferner eine Bilddatenverarbeitungseinrichtung zum Verarbeiten eines Bilddatensatzes, wobei die Bilddatenverarbeitungseinrichtung zum Ausführen eines erfindungsgemäßen Verfahrens eingerichtet ist.

Die Erfindung betrifft ferner ein Computerprogrammprodukt, umfassend Befehle, die bei der Ausführung der Befehle durch einen Computer diesen veranlassen, die Schritte des erfindungsgemäßen Verfahrens auszuführen.

Die Erfindung betrifft ferner ein computerlesbares Speichermedium, umfassend Befehle, die bei der Ausführung der Befehle durch einen Computer diesen veranlassen, die Schritte des erfindungsgemäßen Verfahrens auszuführen.

Bildgebungsvorrichtung kann beispielsweise die Bilddatenverarbeitungseinrichtung aufweisen. Die Bildgebungsvorrichtung kann beispielsweise einen ersten Bildgebungsrohdatensatz mit einer ersten Strahlung, die ein erstes Spektrum und/oder eine erste charakteristische Energie aufweist, akquirieren. Die Bildgebungsvorrichtung kann beispielsweise einen zweiten Bildgebungsrohdatensatz mit einer zweiten Strahlung, die ein zweites Spektrum und/oder eine zweite charakteristische Energie aufweist, akquirieren. Der erste Bilddatensatz kann beispielsweise basierend auf dem ersten Bildgebungsrohdatensatz und basierend auf dem zweiten Bildgebungsrohdatensatz mittels der Bildgebungsvorrichtung rekonstruiert werden. Die Bildgebungsvorrichtung kann beispielsweise ein Dual-Energie-Computertomographiegerät und/oder ein Multi-Energie-Computertomographiegerät sein. Die Bildgebungsvorrichtung kann beispielsweise ein Dual-Source-Computertomographiegerät und/oder ein Multi-Source-Computertomographiegerät sein. Die Bildgebungsvorrichtung kann beispielsweise mehrere unterschiedliche Filter zum Filtern der Strahlung aufweisen.

Der erste Bilddatensatz kann beispielsweise einen Bildpunkt aufweisen. Der Bildpunkt kann beispielsweise eine Positionsinformation und ein Bildwerte-Tupel aufweisen. Die Positionsinformation kann beispielsweise die Position eines Volumenelements eines abzubildenden Bereichs eines Objekts betreffen.

Das Erfassen des ersten Bilddatensatzes kann eine Akquisition des ersten Bilddatensatzes mittels einer Bildgebungsvorrichtung umfassen. Alternativ kann das Erfassen des ersten Bilddatensatzes ein Laden des ersten Bilddatensatzes aus einer Bilddatenbank umfassen.

Das Verarbeiten des ersten Bilddatensatzes kann insbesondere mittels eines Algorithmus, der auf einem Computer ausgeführt wird, erfolgen. Der Algorithmus kann beispielsweise als Eingangsparameter den ersten Bilddatensatz umfassen. Der Algorithmus kann beispielsweise als Ausgangswert den zweiten Bilddatensatz umfassen.

Das Bereitstellen des zweiten Bilddatensatzes kann eine Ausgabe des zweiten Bilddatensatzes für einen Benutzer mittels einer Ausgabevorrichtung und/oder ein Abspeichern des zweiten Bilddatensatzes in einer Datenbank umfassen.

Der Zerlegungs-Bilddatensatz kann insbesondere ein Basismaterialbild sein und/oder umfassen. Insbesondere kann ein Wert eines Bildpunkts des Zerlegungs-Bilddatensatzes basierend auf dem zweiten Bildwerte-Tupel ermittelt werden.

Das Bereitstellen des Zerlegungs-Bilddatensatzes kann eine Anzeige des Zerlegungs-Bilddatensatzes für einen Benutzer mittels einer Ausgabevorrichtung und/oder ein Abspeichern des Zerlegungs-Bilddatensatzes in einer Datenbank umfassen.

Ein Bildwerte-Tupel kann beispielsweise einem Volumenelement eines abzubildenden Bereichs eines Objekts zugeordnet sein. Ein Bildwerte-Tupel kann mehrere Bildwerte aufweisen. Ein Bildwert kann beispielsweise ein Schwächungswert, ein Computertomographie-Wert, ein Intensitätswert, ein Hounsfield-Wert, ein Grauwert, eine Konzentration, ein Dichte oder ähnliches sein und/oder darstellen.

Das Bildwerte-Tupel kann insbesondere in Bezug auf einen Basiskomponenten-Satz dargestellt werden. Der Basiskomponenten-Satz kann insbesondere ein Basismaterialien-Satz sein und/oder einen Basismaterialien-Satz aufweisen. Der Basiskomponenten-Satz kann mehrere Basiskomponenten aufweisen. Insbesondere kann der Basismaterialien-Satz ein erstes Basismaterial und/oder ein zweites Basismaterial aufweisen. Beispielsweise kann jeder Basiskomponente des Basiskomponenten-Satzes ein Bildwert des Bildwerte-Tupels zugeordnet sein. Insbesondere kann der Bildwert als ein Maß für das Vorhandensein und/oder für die Relevanz der Basiskomponente, welcher der Bildwert zugeordnet ist, in dem Volumenelement, dem das Bildwerte-Tupel zugeordnet ist, verstanden werden.

Eine Basiskomponente kann beispielsweise ein Basismaterial, eine Basisenergie, ein Basisspektrum, ein Basisparameter oder Kombinationen daraus sein.

Ein Basismaterial kann beispielsweise ein Kontrastmittel, insbesondere lod, Gewebe, insbesondere Weichgewebe, Blut, Wasser, Knochen, Knorpel, Fett und ähnliches sein. Ein Basismaterial kann beispielsweise eine Kombination und/oder eine Mischform aus mehreren Materialen sein. Dabei können zusätzliche Annahmen, insbesondere Randbedingungen, in Bezug auf die Dichte der mehreren Materialien berücksichtigt werden. Ein Bildwert, der einem Basismaterial zugeordnet ist, kann beispielsweise einer Konzentration, einem Volumenanteil, einem Massenanteil oder einer Dichte des Basismaterials entsprechen.

Ein Basiskomponenten-Satz kann beispielsweise ein Kontrastmittel, insbesondere lod, als eine erste Basiskomponente und eine Mischform aus Blut und Knochen als zweite Basiskomponente aufweisen. Entsprechend kann das Bildwerte-Tupel eine Kontrastmittel-Konzentration als ersten Bildwert und eine Konzentration der Mischform als zweiten Bildwert aufweisen. Die Konzentration der Mischform, die im Folgenden auch als Mischform-Konzentration bezeichnet wird, kann beispielsweise derart definiert sein, dass bei einer ersten Mischform-Konzentration die Konzentration eines ersten Materials der Mischform gleich Null ist und dass bei einer zweiten Mischform-Konzentration die Konzentration eines zweiten Materials der Mischform gleich Null ist. Statt der Mischform-Konzentration kann auch die Konzentration eines Materials der Mischform verwendet werden, wobei eine gegebene Konzentration des anderen Materials der Mischform als konstanter Beitrag berücksichtigt wird. Details zu Basismaterialien sind dem Fachmann insbesondere im Kontext der Zwei-Material-Zerlegung sowie der Drei-Material-Zerlegung bekannt.

Eine Basisenergie kann beispielsweise die Energie einer Strahlung, mit der der Bildwert aufgenommen wurde, sein. Ein Basisspektrum kann beispielsweise das Spektrum einer Strahlung, mit der der Bildwert aufgenommen wurde, sein.

Ein Bildwert, der einer Basisenergie und/oder einem Basisspektrum zugeordnet ist, kann beispielsweise ein Schwächungswert, ein Computertomographie-Wert, ein Intensitätswert oder ähnliches sein. Unterschiedliche Basisenergien und/oder unterschiedliche Basisspektren sind in der Computertomographie beispielsweise durch unterschiedliche Strahlungsquellen, unterschiedliche Röhrenspannungen und/oder unterschiedliche Filter realisierbar. Details zu Basisenergien bzw. Basisspektren sind dem Fachmann insbesondere im Kontext von Dual- bzw. Multi-Energie-Computertomographie, Dual- bzw. Multi-Source-Computertomographie, Dual- bzw. Multi-Spektren-Computertomographie bekannt.

Unter einer multispektralen Computertomographie kann insbesondere eine Multi-Energie-Computertomographie, insbesondere eine Dual-Energie-Computertomographie, eine Multi-Source-Computertomographie, insbesondere eine Multi-Source-Computertomographie, eine Multi-Spektren-Computertomographie, insbesondere eine Multi-Source-Computertomographie, oder ähnliches oder eine Kombination davon verstanden werden.

Ein Basisparameter kann beispielsweise eine Art einer Wechselwirkung, beispielsweise mittels eines photoelektrischen Effekts und/oder mittels einer Compton-Streuung, von Strahlung mit einem Objekt betreffen. Ein Basisparameter kann beispielsweise eine geeignete Kombination aus einer Elektronendichte, einer Kernladungszahl, einer Massezahl und/oder weiteren, physikalischen und/oder chemischen Parametern, die das Volumenelement betreffen sein. Ein Basiskomponenten-Satz kann beispielsweise einen ersten Basisparameter, der den photoelektrischen Effekt betrifft als erste Basiskomponente aufweisen und einen zweiten Basisparameter, der die Compton-Streuung betrifft, als zweite Basis-Komponente aufweisen. Entsprechend kann der erste Bildwert ein Schwächungswert, der ein Maß für die Relevanz des photoelektrischen Effekts in dem Volumenelement ist, und der zweite Bildwert ein Schwächungswert, der ein Maß für die Relevanz der Compton-Streuung in dem Volumenelement ist, sein.

Ein Basiskomponenten-Satz für eine Darstellung des Bildwerte-Tupels kann aus einer Vielzahl möglicher Basiskomponenten-Sätze gewählt werden. In unterschiedlichen Basiskomponenten-Sätzen können die Bildwerte des Bildwerte-Tupels unterschiedlich sein und/oder unterschiedliche Bedeutungen aufweisen. Zwischen den Darstellungen des Bildwerte-Tupels in unterschiedlichen Basiskomponenten-Sätzen kann gewählt und/oder transformiert werden, ohne den Bereich der Erfindung zu verlassen, welcher durch die Ansprüche vorgegeben ist.

Das offenbarte Verfahren zielt auf eine Korrektur der metallbedingten Strahlaufhärtungsartefakte durch Ausnutzung von spektralen Informationen ab. Dies ist anwendbar, wenn energieaufgelöste CT-Messungen desselben Patienten/Objekts zur Verfügung stehen, beispielsweise bei Dual-Energy-CT (Dual Source, Twin Beam, Dual Layer, kV-Switching) oder bei Photon-Counting-CT.

Aufbauend auf dem Schema von DE 10 2016 204 709 A1 wird eine spektrale Rekonstruktion inklusive Strahlaufhärtungskorrektur mit einer generalisierten Basismaterialzerlegung vorgeschlagen.

Hiermit wird ein Verfahren zur Bildrekonstruktion offenbart, das Verfahren umfassend
- eine Aufnahme von *M* spektralen Datensätzen und Rekonstruktion von *M* ersten Bilddaten für jedes Spektrum, wie beispielsweise in DE 10 2016 204 709 A1 beschrieben,
- eine Durchführung einer Materialzerlegung in *M* vorgegebenen Materialien in Materialanteile *b*₁, ... , *b_{M},* mit den folgenden Merkmalen:
- Zerlegung des Wertebereichs der möglichen lokalen Schwächung im Bildraum (= Bildpunktwerte) in mehrere, *K,* Unterbereiche, z.B. durch Schwellwerte der Bildpunktwerte im ersten Spektrum, oder einer gewichteten Mischung (z. B. Linearkombination) aller Spektren.
- Anwendung eines für jeden der *K* Unterbereiche individuellen Zerlegungsmodells der *M* spektralen Bilddatensätze in die *M* gewählten Materialien.

Danach liegt pro Bildpunkt ein Vektor der Anteile der *M* Basismaterialien vor,
- eine Verwendung der so ermittelten Basismaterialanteile von *M* - 1 Materialien daraus selektierten Materialen für die Korrektur der *M* spektralen Datensätze, wie beispielsweise in DE 10 2016 204 709 A1 beschrieben.

Die Basismaterialzerlegung kann in einfachster Weise als (affin) lineare Dekomposition mithilfe einer vom Unterbereich *k =* 1, ... , *K* abhängigen *M* × *M* Matrix ***D***^{(*k*)} realisiert werden, $\left(\begin{matrix}{b}_{1} \\ ⋮ \\ {b}_{M}\end{matrix}\right) = {D}^{\left(k\right)} ⋅ \left(\left(\begin{matrix}{S}_{1} \\ ⋮ \\ {S}_{M}\end{matrix}\right)-\left(\begin{matrix}{o}_{1}^{\left(k\right)} \\ ⋮ \\ {o}_{M}^{\left(k\right)}\end{matrix}\right)\right)$

Komplexere Zerlegungen, d.h. allgemein eine Abbildung ***S*** → ***b***, können selbstverständlich ebenso verwendet werden.

Ebenso denkbar ist eine größere Zahl von Spektren, *M',* als Basismaterialien, *M,* d.h. *M' > M.* In diesem Fall ist das System überbestimmt, jedoch eine Materialzerlegung trotzdem möglich, z.B. mit Transformationsmatrizen ***D***^{(*k*)} der Dimension *M* × *M'.*

In diesem Fall werden bei der Korrektur der spektralen Datensätze abweichend von DE 10 2016 204 709 A1 entsprechend mehr (*M'*) korrigierte spektrale Datensätze erzeugt.

Die Zunahme der Sichtbarkeit von Artefakten in abgeleiteten spektralen Ergebnissen hängt hauptsächlich mit zwei Eigenschaften deren Berechnung zusammen:
Es seien *Sᵢ* die primären spektralen Daten für die Spektren *i* = 1, ..., *N.* Abgeleitet spektrale Ergebnisse sind typischerweise von der Form *R* = Σ*ᵢ wᵢSᵢ,* wobei Gewichte betraglich größer als 1 werden können, d.h. |*wᵢ*| > 1, und Σ*ᵢ wᵢ* = 1 (z.B. monoenergetische Bilder) oder Σ*ᵢ wᵢ* = 0 (bestimmte Basismaterialien).

Abgeleitete spektrale Ergebnisse werden gegenüber den primären spektralen Daten stärker statistisch optimiert, d.h. Rauschen reduziert. Dadurch steigt im Allgemeinen auch das Signal-zu-Rausch-Verhältnis von Artefakten.

Hiermit wird ein Verfahren zur Artefakt-Korrektur offenbart, das Verfahren umfassend:
- eine Transformation der primären spektralen Daten durch eine affin lineare Transformation, beispielsweise in der Form *S'ⱼ =* Σ*ᵢ AⱼᵢSᵢ + oⱼ* und/oder mit der Eigenschaft, dass die insbesondere quadratische Matrix *A* invertierbar ist,
- eine Rauschreduktion auf den transformierten spektralen Daten, beispielsweise auf den transformierten spektralen Daten *S'ⱼ*, wobei sich rauschreduzierte Daten, beispielsweise rauschreduzierte Daten *Ŝ'ⱼ*, ergeben,
- eine Bestimmung eines Artefakt-Bildes *E'ⱼ* pro Freiheitsgrad, *j,* derart, dass *Ŝ'ⱼ - E'ⱼ* jeweils ein Ergebnis mit reduzierten Artefakten darstellt,
- eine Transformation der Artefakt-Bilder in den Konfigurationsraum der primären spektralen Daten, *Eᵢ* = ∑*ᵢ*(*A*⁻¹)*ᵢⱼE'ⱼ,*
- eine Korrektur der primären spektralen Daten durch Subtraktion der transformierten Artefakt-Bilder, *Sⱼ* - *Eⱼ*.

Die Reihenfolge der Transformation und der Rauschreduktion kann vertauscht werden. Beispielsweise kann zuerst die Rauschreduktion auf den primären Daten *Sᵢ* und anschließend die affin lineare Transformation durchgeführt werden.

Eine geeignete Transformation ist zum Beispiel gegeben durch $A = \left(\begin{matrix}1 & 0 \\ - 1 & 1\end{matrix}\right) für N = 2 , A = \left(\begin{matrix}1 & 0 & 0 \\ - 1 & 1 & 0 \\ - 1 & 0 & 1\end{matrix}\right) für N = 3 , …$

Dadurch wird ein Spektrum der primären Daten ausgezeichnet (hier ohne Beschränkung der Allgemeinheit das erste), vorteilhaft dadurch charakterisiert, dass es das geringste Rauschen aller Primärspektren aufweist. Die anderen Spektren werden hier in die jeweilige Differenz zu dem ersten Spektrum transformiert. Motiviert ist diese Wahl dadurch, dass in abgeleiteten spektralen Ergebnissen oft Differenzen zwischen spektralen Kanälen mit hohem Gewicht auftreten. Durch den Offset *oⱼ* kann der Werte-Bereich in den typischen Bereich der Artefakt-Erkennung gebracht werden.

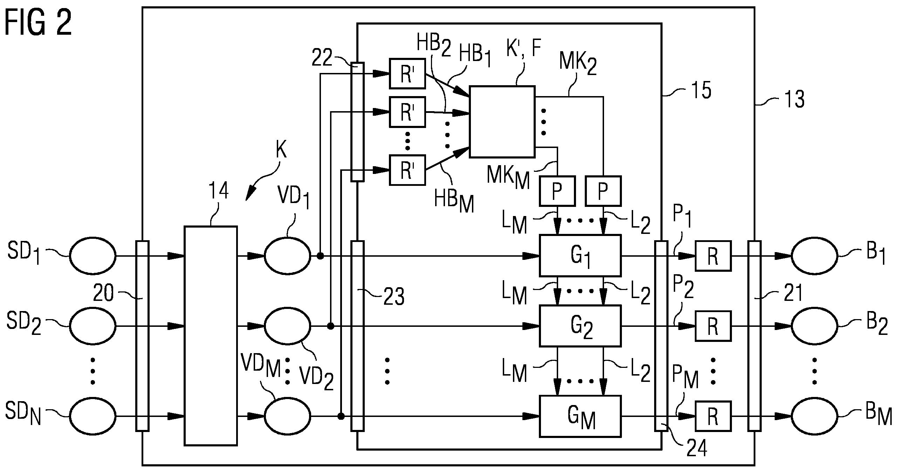

Fig. 2 zeigt ein Flussdiagramm des Verfahrens zur Artefakt-Korrektur für exemplarisch *N* = 2. In vorteilhafter Weise ist die Rauschreduktion morphologisch synchronisiert, wie beispielsweise in DE 10 2019 210 355 A1 erläutert, da die Daten dasselbe Objekt/Subjekt abbilden, aber aufgrund der unterschiedlichen Spektren unterschiedliche CT-Werte bzw. Kontraste zeigen.

Das Verfahren zur Artefakt-Korrektur ist nicht auf die Entfernung von Ringartefakten beschränkt, für diesen Zweck aber besonders relevant. Die Art der Akquisition der primären spektralen Daten ist unerheblich. Es können Dual Source Dual Energy, Multi-Layer Detektoren, kV Switching, oder insbesondere Schwellen oder Bins von photonenzählenden Detektoren verwendet werden.

Das vorgestellte Verfahren berücksichtigt mehr Materialien als aufgrund der Zahl der Freiheitsgrade durch die spektrale Information eigentlich zur Verfügung stehen. Dies geschieht mithilfe der Überblendung zwischen verschiedenen Materialsystemen wie z.B. lokale Höhe des Schwächungskoeffizienten. Auch wenn dadurch nicht unmittelbar auf das "korrekte" Materialsystem, das das tatsächlich vorhandene Metall/Legierung beschreibt, geschlossen werden kann, so sind die Surrogate doch erheblich näher an einem generischen, gewählten Basismaterialsystem und damit die Ergebnisse im Hinblick auf die Strahlaufhärtungskorrektur besser.

Ziel der Erfindung ist die Reduktion dieser Artefakte unter Ausnutzung spektraler Information der CT-Messdaten. Die Annahme, dass jedes andere Material sich als Linearkombination der Basismaterialien darstellen lässt, ist im Allgemeinen nur approximativ gültig, insbesondere wenn das andere Material nicht als "kleine Störung", sondern in hohen Konzentrationen vorkommt. Das ist bei Metallen der Fall, weswegen die Qualität der Korrektur bei N(Freiheitsgrade) = N(Spektren) abnimmt.

Das vorgeschlagene Verfahren erhöht das Signal-zu-Rausch-Verhältnis der Artefakte vor der Detektion spezifisch so, dass deren spätere Sichtbarkeit in den abgeleiteten Ergebnissen besser berücksichtigt wird. Dies löst das Problem, dass die Detektion Artefakte "übersieht", weil sie in der primären Daten-Repräsentation zu wenig signifikant sind.

Gemäß einem Aspekt der Erfindung ist die Bildgebungsvorrichtung eine medizinische Bildgebungsvorrichtung. Gemäß einem Aspekt der Erfindung ist die Bildgebungsvorrichtung aus der Gruppe gewählt, welche aus einem C-Bogen-Röntgengerät, einem Computertomographiegerät (CT-Gerät), einem Einzelphotonen-Emissions-Computertomographiegerät (SPECT-Gerät), einem Positronen-Emissions-Tomographiegerät (PET-Gerät), einem Magnetresonanztomographiegerät (MRT-Gerät) und Kombinationen davon besteht. Insbesondere kann die Bildgebungsvorrichtung ein Röntgengerät, ein Ultraschallgerät und ähnliches aufweisen. Die Bildgebungsvorrichtung kann ferner eine Kombination von mehreren Bildgebungs- und/oder Bestrahlungsmodalitäten sein. Dabei kann eine Bestrahlungsmodalität beispielsweise ein Bestrahlungsgerät zur therapeutischen Bestrahlung aufweisen.

Gemäß einem Aspekt der Erfindung wird der erste Bilddatensatz mittels der Bildgebungsvorrichtung erzeugt und/oder bereitgestellt. Gemäß einem Aspekt der Erfindung ist die Bildgebungsvorrichtung dazu ausgebildet, den ersten Bilddatensatz zu erzeugen und/oder bereitzustellen.

Eine Ausführungsform der Erfindung sieht vor, dass die erfindungsgemäße Bilddatenverarbeitungseinrichtung und/oder eine oder mehrere Komponenten der erfindungsgemäßen Bilddatenverarbeitungseinrichtung zumindest teilweise in Form von Software auf einem Prozessorsystem realisiert sind. Insbesondere das Erfassungsmodul, das Verarbeitungsmodul, das Bereitstellungsmodul, das Erzeugungsmodul, das Auswahlmodul und das Ermittlungsmodul können jeweils eine Komponente der erfindungsgemäßen Bilddatenverarbeitungseinrichtung bilden. Eine Ausführungsform der Erfindung sieht vor, dass die erfindungsgemäße Bilddatenverarbeitungseinrichtung und/oder eine oder mehrere Komponenten der erfindungsgemäßen Bilddatenverarbeitungseinrichtung zumindest teilweise in Form von softwareunterstützter Hardware, beispielsweise FPGAs, einem Prozessorsystem oder dergleichen, realisiert sind.

Ein Datentransfer zwischen Komponenten der Bilddatenverarbeitungseinrichtung kann z. B. mittels einer geeigneten Schnittstelle erfolgen. Eine Ausführungsform der Erfindung sieht vor, dass Schnittstellen zum Datentransfer an und/oder von Komponenten der erfindungsgemäßen Bilddatenverarbeitungseinrichtung zumindest teilweise in Form von Software realisiert sind. Insbesondere können die Schnittstellen Zugriffsmöglichkeiten auf geeignete Speicherbereiche, in denen Daten geeignet zwischengelagert, aufgerufen und aktualisiert werden können, aufweisen. Die Schnittstellen können auch als hardwaremäßig aufgebaute Schnittstellen ausgebildet sein, die durch geeignete Software angesteuert werden.

Eine weitgehend softwaremäßige Realisierung der erfindungsgemäßen Bilddatenverarbeitungseinrichtung hat den Vorteil, dass auch schon bisher verwendete Bildgebungsvorrichtungen und/oder Computer durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches in eine Speichereinrichtung eines Computers ladbar ist, wobei mit dem Computerprogramm die Schritte eines erfindungsgemäßen Verfahrens ausgeführt werden, wenn das Computerprogramm auf dem Computer ausgeführt wird. Ein solches Computerprogrammprodukt kann neben dem Computerprogramm zusätzliche Software-Komponenten, z. B. eine Dokumentation, und/oder Hardware-Komponenten, z. B. einen Hardware-Schlüssel (Dongle etc.) zur Nutzung der Software, umfassen.

Zum Transport des Computerprogramms und/oder zur Speicherung des Computerprogramms an oder in einem Computer kann ein computerlesbares Medium, beispielsweise ein Memorystick, eine Festplatte oder ein sonstiger transportabler oder fest eingebauter Datenträger, dienen, auf welchem ein Computerprogramm gespeichert ist, welches in eine Speichereinrichtung eines Computers ladbar ist, wobei mit dem Computerprogramm die Schritte eines erfindungsgemäßen Verfahrens ausgeführt werden, wenn das Computerprogramm auf dem Computer ausgeführt wird. Eine Ausführungsform der Erfindung sieht vor, dass die erfindungsgemäße Bildgebungsvorrichtung und/oder die erfindungsgemäße Bilddatenverarbeitungseinrichtung einen Computer aufweist. Der Computer kann jeweils ein Prozessorsystem aufweisen, welches z. B. einen Mikroprozessor oder mehrere zusammenarbeitende Mikroprozessoren aufweist.

Im Rahmen der Erfindung können Merkmale, welche in Bezug auf unterschiedliche Ausführungsformen und/oder unterschiedliche Anspruchskategorien (Verfahren, Bilddatenverarbeitungseinrichtung usw.) beschrieben sind, zu weiteren Ausführungsformen kombiniert werden. Insbesondere sind die in Bezug auf das erfindungsgemäße Verfahren beschriebenen Merkmale, Vorteile und Ausführungsformen auch auf die erfindungsgemäße Bilddatenverarbeitungseinrichtung, das erfindungsgemäße Computerprogrammprodukt und das erfindungsgemäße computerlesbare Medium zu übertragen und umgekehrt. Mit anderen Worten können die gegenständlichen Ansprüche auch mit den Merkmalen, die in Zusammenhang mit einem Verfahren beschrieben oder beansprucht sind, weitergebildet sein. Funktionale Merkmale eines erfindungsgemäßen Verfahrens können dabei durch entsprechend ausgebildete Komponenten bzw. Module der erfindungsgemäßen Bilddatenverarbeitungseinrichtung ausgeführt werden.

Bei dem beschriebenen Verfahren und der beschriebenen Bilddatenverarbeitungseinrichtung handelt es sich lediglich um Ausführungsformen der Erfindung. Die Erfindung kann vom Fachmann variiert werden, ohne den Bereich der Erfindung zu verlassen, soweit er durch die Ansprüche vorgegeben ist.

Die Verwendung der unbestimmten Artikel "ein" bzw. "eine" schließt nicht aus, dass die betreffenden Merkmale auch mehrfach vorhanden sein können. Die Verwendung des Ausdrucks "aufweisen" schließt nicht aus, dass die mittels des Ausdrucks "aufweisen" verknüpften Begriffe identisch sein können. Beispielsweise weist die medizinische Bildgebungsvorrichtung die medizinische Bildgebungsvorrichtung auf. Es ist möglich, dass ein Modul mehrere, räumlich voneinander separierte Teilmodule aufweisen kann.

Die Verwendung von Ordnungszahlwörtern (erste, zweite, dritte etc.) in der Bezeichnung von Merkmalen dient im Kontext der vorliegenden Anmeldung vor allem der besseren Unterscheidbarkeit der unter Verwendung von Ordnungszahlwörtern bezeichneten Merkmale. Das Nichtvorhandensein eines Merkmals, welches durch eine Kombination eines gegebenen Ordnungszahlworts und eines Begriffs bezeichnet wird, schließt nicht aus, dass ein Merkmal vorhanden sein kann, welches durch eine Kombination eines dem gegebenen Ordnungszahlwort nachfolgenden Ordnungszahlworts und des Begriffs bezeichnet wird.

Der Ausdruck "basierend auf" kann im Kontext der vorliegenden Anmeldung insbesondere im Sinne des Ausdrucks "unter Verwendung von" verstanden werden. Insbesondere schließt eine Formulierung, der zufolge ein erstes Merkmal basierend auf einem zweiten Merkmal erzeugt (alternativ: ermittelt, bestimmt etc.) wird, nicht aus, dass das erste Merkmal basierend auf einem dritten Merkmal erzeugt (alternativ: ermittelt, bestimmt etc.) werden kann.

Die Erfindung wird im Folgenden unter Hinweis auf die beigefügten Figuren anhand von Ausführungsbeispielen noch einmal näher erläutert. Die Darstellung in den Figuren ist schematisch und stark vereinfacht sowie nicht zwingend maßstabsgetreu. Es zeigen:
- FIG 1: eine grob schematische Darstellung eines Ausführungsbeispiels eines erfindungsgemäßen Computertomographiesystems,
- FIG 2: eine schematische Darstellung eines Ausführungsbeispiels einer erfindungsgemäßen Bildrekonstruktionseinrichtung mit einem Blockschema eines Ausführungsbeispiels eins erfindungsgemäßen Verfahrens zur Bildrekonstruktion,
- FIG 3: eine schematische Darstellung eines Ausführungsbeispiels erfindungsgemäßen ersten iterativen Verfahrens zur Bildrekonstruktion und
- FIG 4: eine schematische Darstellung eines Ausführungsbeispiels erfindungsgemäßen zweiten iterativen Verfahrens zur Bildrekonstruktion.
- FIG 5: eine Darstellung der Abbildung, die auf das erste Bildwerte-Tupel und das zweite Bildwerte-Tupel angewendet wird.
- FIG 6: ein Flussdiagramm eines Verfahrens zur Rauschreduktion.

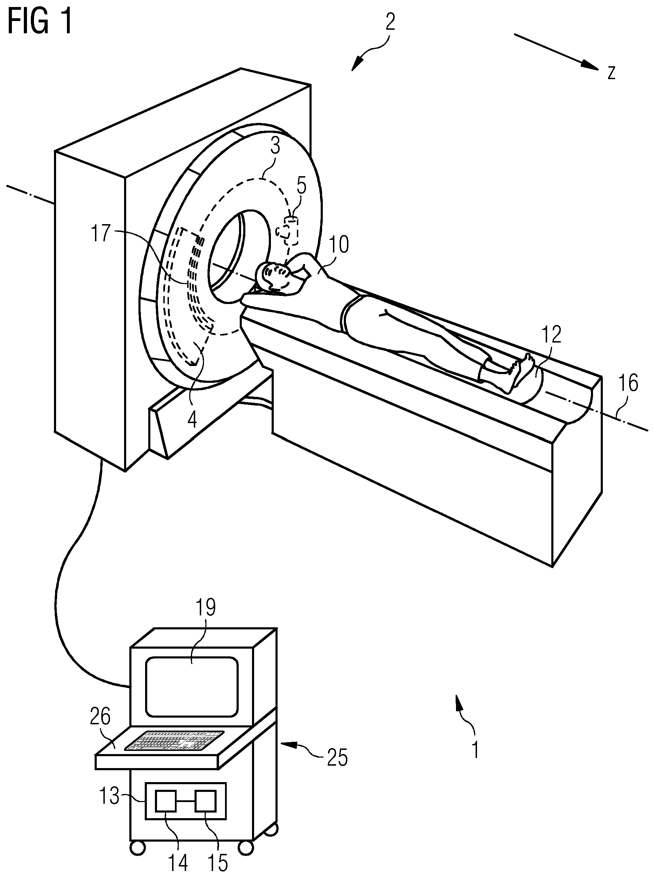

In FIG 1 ist beispielhaft und grob schematisch ein erfindungsgemäßes Computertomographiesystem 1 gezeigt, welches ein Benutzerterminal 25 und ein Computertomographiegerät 2 umfasst. Das Computertomographiesystem 1 ist zur Ausführung des erfindungsgemäßen Verfahrens zur Bildrekonstruktion ausgebildet. Das Computertomographiegerät 2 umfasst einen Patiententisch 12 zur Lagerung eines Patienten 1 als Untersuchungsobjekt, welcher entlang einer Systemachse 16 verstellbar ist. Die Systemachse 16 wird im Folgenden auch als z-Achse bezeichnet, die mit dem Patienten 10 in das Messfeld verstellbar ist. Es umfasst ferner eine Gantry 3 mit einer um die Systemachse 16 drehbar gelagerten Quelle-Detektor-Anordnung 4, 5. Die Quelle-Detektor-Anordnung 4, 5 weist eine Röntgenstrahlungsquelle 5 und einen quantenzählenden Detektor 4 auf, die einander gegenüberliegend so ausgerichtet sind, dass im Betrieb eine von dem Fokus der Röntgenstrahlungsquelle 5 ausgehende Röntgenstrahlung auf den Detektor 4 trifft. Der Detektor 4 ist zur ortsaufgelösten Erfassung der Röntgenstrahlung in einzelne Pixel 17 strukturiert, die zu mehreren Detektorzeilen angeordnet sind. Derzeit werden Detektoren 4 eingesetzt, die über insgesamt 64 oder mehr Zeilen verfügen und eine Ortsauflösung im Submillimeterbereich aufweisen. Zu jeder Projektion erzeugt der Detektor 4 einen Satz von Projektionsdaten. Die Projektionsdaten repräsentieren dabei die Schwächungswerte sämtlicher Pixel 17 einer durch den Patienten 1 geschwächten Röntgenstrahlung. Sie werden je nach ihrer Energie in separaten Bins der Pixel 17 des Detektors 4 erfasst wird. Jeweils die Anteile der Projektionsdaten sämtlicher Projektionen, die mit gleicher Energie in entsprechenden Bins erfasst wurden, sind hier ein Spektral-Rohdatensatz SD₁, SD₂, ..., SD_{N}. Die Spektral-Rohdatensätze SD₁, SD₂, ..., SD_{N} werden an das Benutzerterminal 25 mit einer Bildrekonstruktionseinrichtung 13 weitergeleitet und mittels eines erfindungsgemäßen Verfahrens zu einem resultierenden Bild verrechnet, welches z. B. auf einer Anzeigeeinheit 19 darstellbar ist und/oder welches in einem Speicher hinterlegt und/oder an andere Systeme versandt werden kann. Dafür umfasst die Bildrekonstruktionseinrichtung 13 ein Kombinationsmodul 14 und ein Optimierungsmodul 15. Das Benutzerterminal 25 umfasst des Weiteren eine Tastatur 26 als Eingabegerät, mit dem ein Bediener gegebenenfalls Werte für Parameter bei der Bildrekonstruktion einstellen kann.

Ein solches Computertomographiegerät 2 wird bekanntermaßen zur 3D-Bildrekonstruktion eingesetzt. Zur Aufnahme eines Bildes von einem Untersuchungsgebiet (Region of Interest) werden bei Rotation der Quelle-Detektor-Anordnung 4, 5 Projektionsdaten aus einer Vielzahl von unterschiedlichen Projektionsrichtungen in energieaufgelöst in Bins als Spektral-Rohdatensätze SD₁, SD₂, ..., SD_{N} erfasst. Im Fall einer Spiralabtastung erfolgt während einer Rotation der Quelle-Detektor-Anordnung 4, 5 beispielsweise gleichzeitig eine kontinuierliche Verstellung des Patiententisches 12 in Richtung der Systemachse 16. Die Röntgenstrahlungsquelle 5 und der Detektor 4 bewegen sich bei dieser Art der Abtastung somit auf einer Helixbahn um den Patienten 10.

FIG 2 zeigt beispielhaft eine schematische Darstellung einer erfindungsgemäßen Bildrekonstruktionseinrichtung 13 mit einem Blockschema eins erfindungsgemäßen Verfahrens zur Bildrekonstruktion. Die Bildrekonstruktionseinrichtung 13 weist ein Kombinationsmodul 14 und ein Optimierungsmodul 15 sowie eine Eingangsschnittstelle 20 und eine Ausgangsschnittstelle 21 auf. Die Eingangsschnittstelle 20 empfängt eine Anzahl von *N* vom Computertomographiegerät 2 akquirierten Spektral-Rohdatensätzen SD₁, SD₂, ..., SD_{N} und leitet sie an das Kombinationsmodul 14 weiter. Das Kombinationsmodul 14 führt eine Linearkombination K der Spektral-Rohdatensätze SD₁, SD₂, ..., SD_{N} durch und erzeugt dabei eine Anzahl von *M* virtuellen Rohdatensätzen VD₁, VD₂, ..., VD_{M}. Ab der Erzeugung der virtuellen Rohdatensätzen VD₁, VD₂, ..., VD_{M} bezeichnen die Indices
1, 2, ..., M virtuelle spektrale Kanäle. Sie sind also dem entsprechend kombiniertem Spektrum sowie später auch einem Material zugeordnet.

Bei der Linearkombination K werden die Spektral-Rohdatensätze SD₁, SD₂, ..., SD_{N} für jeweils einen virtuellen Rohdatensatz VD₁, VD₂, ... , VD_{M} mit Koeffizienten, also skalaren Faktoren, multipliziert und folgend addiert, sodass sie nach Wahl des Bedieners definierte mittlere bzw. effektive Energien aufweisen und/oder statistisch möglichst schwach korreliert sind. Die Anzahl *M* ist bevorzugt 3, besonders bevorzugt 2, und damit geringer als die Anzahl *N* der Spektral-Rohdatensätze SD₁, SD₂, ..., SD_{N}, da der Detektor 4 das Spektrum der Röntgenstrahlung meist in mehr Energieschwellen auflöst, als derzeit sinnvoll für die folgende materialabhängige Optimierung der Rekonstruktion genutzt werden können.

Das Optimierungsmodul 15 weist eine erste Eingangsschnittstelle 22 und eine zweite Eingangsschnittstelle 23 sowie eine Ausgangsschnittstelle 24 auf. Die virtuellen Rohdatensätze VD₁, VD₂, ..., VD_{M} werden vom Kombinationsmodul 14 sowohl an die erste Eingangsschnittstelle 22 zur Erstellung von optimierenden Abbildungsfunktionen G₁, G₂, ..., G_{M} als auch an die zweite Eingangsschnittstelle 23 als Argumente für die optimierenden Abbildungsfunktionen G₁, G₂, ..., G_{M} übertragen.

Die von der ersten Eingangsschnittstelle 22 empfangenen virtuellen Rohdatensätze VD₁, VD₂, ..., VD_{M} werden jeweils mittels einer hilfsweisen gefilterten Rückprojektion R' zu Hilfsbilddatensätzen HB₁, HB₂, ..., HB_{M} rekonstruiert. Es folgt eine weitere, optionale Linearkombination K' der Hilfsbilddatensätze HB₁, HB₂, ..., HB_{M} zu neu gewichteten Hilfsbilddatensätzen, die einem Material zugeordnet und beispielsweise wieder statisch möglichst schwach korreliert sind. Einer der Hilfsbilddatensätze HB₁ ist dem Material "Wasser" zugeordnet, das (entsprechend Schritt c) des zuvor beschriebenen Verfahrens) ausgewählt wird. Aus den übrigen *M-1* Hilfsbilddatensätze HB₂, HB₃, ..., HB_{M} werden jeweils voxelweise mittels einer Schwellenfunktion F materialspezifische Karten MK₂, MK₃, ..., MK_{M} berechnet. Diese bilden im Wesentlichen die lokalen Konzentrationen der jeweiligen Materialien ab, können aber auch eine Elektronendichtekarte oder eine Kernladungskarte darstellen.

Folgend (entsprechend Schritt e) des o.g. Verfahrens) werden mittels einer virtuellen Vorwärtsprojektion P unter der gleichen Geometrie wie bei der Akquisition der ursprünglichen Daten aus den materialspezifischen Karten MK₂, MK₃, ..., MK_{M} jeweils Materiallinienintegrale L₂, ..., L_{M} für jedes Material gewonnen. Folgend werden *M* optimierende Abbildungsfunktionen G₁, G₂, ..., G_{M} im Wesentlichen durch eine Linearkombination aus mit den Absorptionskoeffizienten µ₂, µ₃, ..., µ_{M} gewichteten Materiallinienintegrale L₂, ..., L_{M} erzeugt. Zusätzlich dazu wird die erste Komponente der jeweiligen Abbildungsfunktion Gⱼ mit j=1, ..., M die dem ausgewählten Material zugeordnet ist, als Funktion hⱼ von den Materiallinienintegralen L₂, ..., L_{M}, dem Absorptionskoeffizienten µ₁ⱼ des ausgewählten Materials und dem dem jeweiligen virtuellen spektralen Kanal j=1, ..., M zugeordneten virtuellen Rohdatensatz VD₁, VD₂, ..., VD_{M} als Argument wie folgt gebildet: ${G}_{j} \left({VD}_{j}, {L}_{2}, …, {L}_{M}\right) = {h}_{j} \left({VD}_{j}; {L}_{2}, …, {L}_{M}\right) {μ}_{1 j} + {L}_{2} {μ}_{2 j} + {L}_{3} {μ}_{3 j} + …$

Mit den optimierenden Abbildungsfunktionen G₁, G₂, ..., G_{M} werden (entsprechend Schritt f) des o.g. Verfahrens) aus den virtuellen Rohdatensatz VD₁, VD₂, ..., VD_{M} als Argumenten jeweils optimierte synthetische Projektionsdaten P₁, P₂, ..., P_{M} erzeugt. Diese verlassen über die Ausgangsschnittstelle 24 das Optimierungsmodul 15.

Im folgenden Schritt (entsprechend Schritt g) des o.g. Verfahrens) werden die synthetischen Projektionsdaten P₁, P₂, ... , P_{M} jeweils mittels gefilterter Rückprojektion R zu Bilddatensätzen B₁, B₂, ..., B_{M} rekonstruiert. Diese werden an die Ausgangsschnittstelle 21 übermittelt und könne dann beispielsweise auf der Anzeigeeinheit 19 des Benutzerterminals 25 des Computertomographiesystems 1 angezeigt werden oder auch zu belieben anderen Anzeigeeinheiten und/oder Speichereinrichtungen weiterübertragen werden.

In der Regel weisen alle Daten des beschriebenen Verfahrens, also von den Spektral-Rohdaten SD₁, SD₂, ..., SD_{N} bis zu den Bilddaten B₁, B₂, ..., B_{M} und insbesondere auch die Abbildungsfunktionen Gⱼ, eine Abhängigkeit von Parametern des Strahls auf. Parameter des Strahls sind dabei beispielsweise Projektionswinkel, Kanalnummer, Detektorzeile und dergleichen. Dieser Zusammenhang besteht z. B., weil sich das Röntgenspektrum aufgrund eines Formfilters als Funktion des Abstands vom Drehzentrum ändert. Unabhängig von den Parametern des Strahls bleiben selbstverständlich Größen wie die Absorptionskoeffizienten µ.

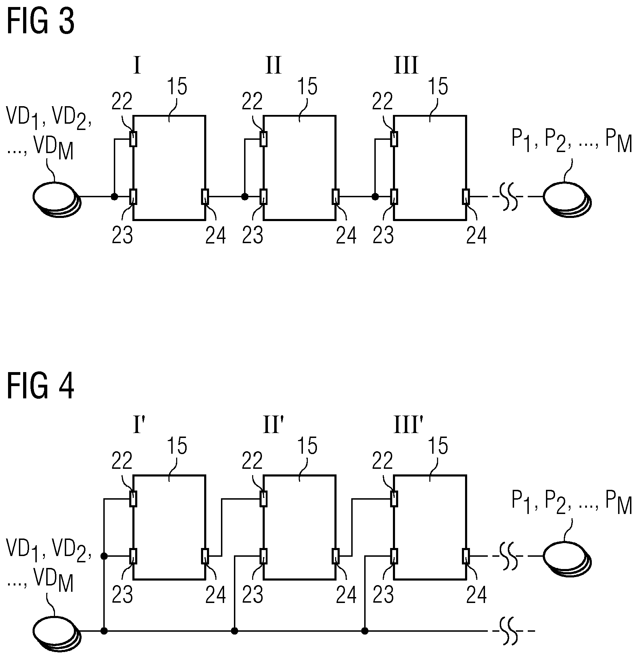

In FIG 3 ist beispielhaft eine schematische Darstellung eine erste Variante eines iterativen Verfahrens zur Bildrekonstruktion dargestellt. Die virtuellen Rohdatensätze VD₁, VD₂, ..., VD_{M} sind in einem vorbereitenden Schritt bereits mittels des Kombinationsmoduls 14 aus den Spektral-Rohdatensäten SD₁, SD₂, ..., SD_{N} kombiniert worden (hier nicht dargestellt). Aus ihnen werden in einer beliebigen Anzahl von Optimierungsdurchläufen I, II, III, ... optimierte synthetische Projektionsdatensätze P₁, P₂, ..., P_{M} erzeugt. Dafür wird ein Optimierungsmodul, welches besonders bevorzugt als Software-Modul ausgestaltet ist, entsprechend mehrfach durchlaufen. Hier ist anschaulich als alternative Ausführung eine entsprechende Anzahl von Optimierungsmodulen 15 hintereinandergeschaltet dargestellt. Das erste Optimierungsmodul 15 empfängt die virtuellen Rohdatensätze VD₁, VD₂, ..., VD_{M} an beiden Eingangsschnittstellen 22, 23. Folgend berechnet es synthetische Projektionsdatensätze P₁, P₂, ..., P_{M} und leitet diese über seine Ausgangsschnittstelle 24 an beide Eingangsschnittstellen 22, 23 des dahinter angeordneten Optimierungsmoduls 15 weiter. Dieser Vorgang wird eine beliebige Anzahl von Durchläufen wiederholt. Schließlich, d. h. sobald der erwünschte Grad an Optimierung erreicht ist, werden die optimierten synthetischen Projektionsdatensätze P₁, P₂, ..., P_{M} ausgegeben und folgend wie bereits geschildert zu Bilddatensätzen B₁, B₂, ..., B_{M} rekonstruiert (hier nicht dargestellt).

FIG 4 zeigt beispielhaft und schematisch eine zweite Variante eines iterativen Verfahrens zur Bildrekonstruktion. Die FIG 4 ist dabei ähnlich zu FIG 3. Der Unterschied besteht darin, dass lediglich die erste Eingangsschnittstelle 22 des jeweiligen Optimierungsmoduls 15 die optimierten synthetischen Projektionsdatensätze P₁, P₂, ..., P_{M} aus dem vorigen Optimierungsdurchlauf l', II', III',... als virtuelle Rohdatensätze VD₁, VD₂, ..., VD_{M} empfängt. Die zweite Eingangsschnittstelle 23 empfängt demgegenüber immer, d. h. in jedem Durchlauf, die ursprünglichen virtuellen Rohdatensätze VD₁, VD₂, ..., VD_{M}.

Das erfindungsgemäße Verfahren ermöglicht eine flexible Nutzung der Mehrspektren-Information zur Reduktion von Strahlaufhärtungsartefakten bzw. zur Berechnung von monochromatischen Bildern oder Basismaterialbildern. Es vereint also die Vorteile von bild- und rohdatenbasierten Verfahren und vermeidet die jeweiligen Nachteile.

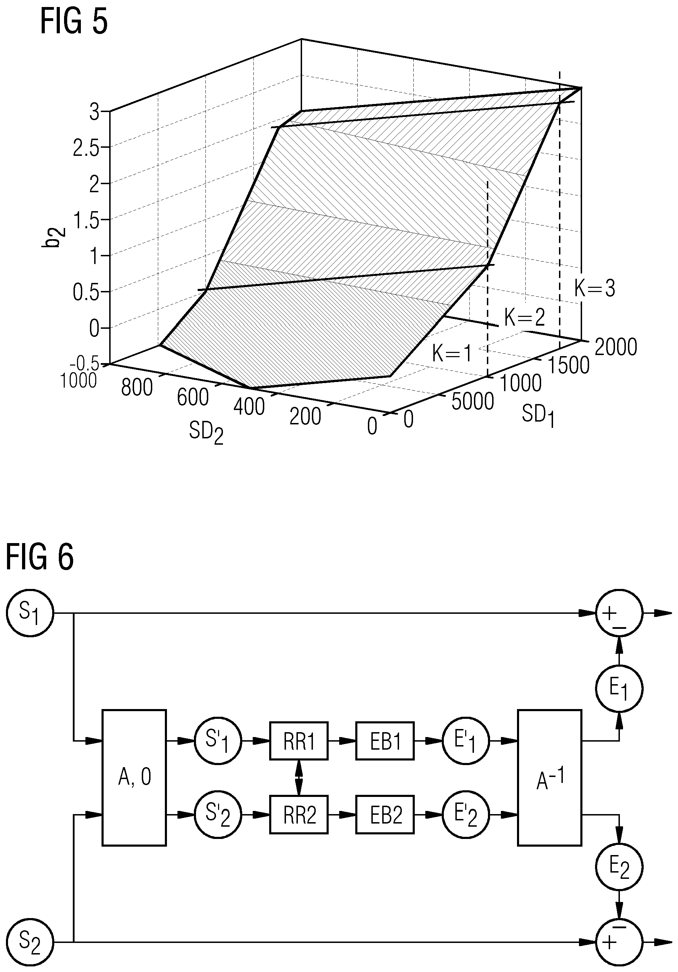

FIG 5 zeigt eine Darstellung der Abbildung, die auf das erste Bildwerte-Tupel und das zweite Bildwerte-Tupel angewendet wird, sowie die Aufteilung in drei Unterbereiche basierend auf einem Maß für die Abschwächung. Die Abbildung ist an den Unterbereichsgrenzen stetig und geht bei K = 3 in die Sättigung.

FIG 6 zeigt ein Flussdiagramm eines Verfahrens zur Rauschreduktion. In den Schritten RR1 und RR2 erfolgt jeweils eine Rauschreduktion. In den Schritten EB1 und EB2 wird jeweils ein Artefakt-Bild ermittelt.

## Patentansprüche

1. Computerimplementiertes Verfahren zum Verarbeiten eines Bilddatensatzes, welcher auf einer multispektralen Computertomographie-Bildgebung basiert und welcher ein erstes Bildwerte-Tupel und ein zweites Bildwerte-Tupel aufweist, wobei das erste Bildwerte-Tupel einem ersten Volumenelement eines abzubildenden Bereichs zugeordnet ist, wobei das zweite Bildwerte-Tupel einem zweiten Volumenelement des abzubildenden Bereichs zugeordnet ist, das Verfahren umfassend:
- ein Berechnen eines ersten Anteils eines Basismaterials, indem eine Abbildung auf das erste Bildwerte-Tupel angewendet wird, wobei der erste Anteil des Basismaterials dem ersten Volumenelement des abzubildenden Bereichs zugeordnet ist, wobei die Abbildung an dem ersten Bildwerte-Tupel eine erste Steigung aufweist, wobei die erste Steigung ungleich Null ist,
- ein Berechnen eines zweiten Anteils des Basismaterials, indem die Abbildung auf das zweite Bildwerte-Tupel angewendet wird, wobei der zweite Anteil des Basismaterials dem zweiten Volumenelement des abzubildenden Bereichs zugeordnet ist, wobei die Abbildung an dem zweiten Bildwerte-Tupel eine zweite Steigung aufweist, wobei die zweite Steigung ungleich Null und ungleich der ersten Steigung ist.

2. Verfahren nach Anspruch 1,
- wobei ein zusammenhängender Teilraum der Bildwerte das erste Bildwerte-Tupel und das zweite Bildwerte-Tupel aufweist,
- wobei die Abbildung an jedem Bildwerte-Tupel des zusammenhängenden Teilraumes stetig ist.

3. Verfahren nach Anspruch 1 oder 2,
- wobei in Bezug auf ein Maß für eine Abschwächung von Röntgenstrahlung die Abschwächung der Röntgenstrahlung durch das zweite Volumenelement stärker ist als die Abschwächung der Röntgenstrahlung durch das erste Volumenelement,
- wobei die Steigung der Abbildung an dem zweiten Bildwerte-Tupel steiler ist als an dem ersten Bildwerte-Tupel.

4. Verfahren nach Anspruch 3,
- wobei der erste Bilddatensatz ein drittes Bildwerte-Tupel aufweist, wobei das dritte Bildwerte-Tupel einem dritten Volumenelement des abzubildenden Bereichs zugeordnet ist,
- wobei in Bezug auf das Maß für die Abschwächung der Röntgenstrahlung die Abschwächung der Röntgenstrahlung durch das dritte Volumenelement stärker ist als die Abschwächung der Röntgenstrahlung durch das zweite Volumenelement,
- wobei eine Steigung der Abbildung an dem dritten Bildwerte-Tupel flacher ist als an dem zweiten Bildwerte-Tupel.

5. Verfahren nach Anspruch 3 oder 4,
- wobei das Maß für die Abschwächung der Röntgenstrahlung ein ausgewähltes Spektrum, insbesondere das Spektrum des ersten Bildwerts der Bildwerte-Tupel, der multispektralen Computertomographie-Bildgebung betrifft.

6. Verfahren nach Anspruch 3 oder 4,
- wobei das Maß für die Abschwächung der Röntgenstrahlung eine Linearkombination, insbesondere in Form einer gewichteten Mischung, von verschiedenen Spektren der multispektralen Computertomographie-Bildgebung zugeordneten Bildwerten betrifft.

7. Verfahren nach einem der Ansprüche 3 bis 6,
- wobei das Maß für die Abschwächung angibt, ob die Abschwächung der Röntgenstrahlung einen vorgegebenen Schwellwert überschreitet und/oder unterschreitet.

8. Bilddatenverarbeitungseinrichtung zum Verarbeiten eines Bilddatensatzes, wobei die Bilddatenverarbeitungseinrichtung zum Ausführen eines Verfahrens nach einem der Ansprüche 1 bis 7 eingerichtet ist.

9. Computerprogrammprodukt, umfassend Befehle, die bei der Ausführung der Befehle durch einen Computer diesen veranlassen, die Schritte des Verfahrens nach einem der Ansprüche 1 bis 7 auszuführen.

10. Computerlesbares Speichermedium, umfassend Befehle, die bei der Ausführung der Befehle durch einen Computer diesen veranlassen, die Schritte des Verfahrens nach einem der Ansprüche 1 bis 7 auszuführen.
